# FASCICULE DE BREVET EUROPEEN

(11) **EP 1 758 533 B1**
(45) Date de publication et mention de la délivrance du brevet: **26.03.2008**
(21) Numéro de dépôt: 05778634.5
(22) Date de dépôt: 17.06.2005
(51) Int. Cl.: A61F 13/06, A61F 13/10

(54) **PROCEDE ET DISPOSITIF POUR ACCELERER LA CROISSANCE DES PHANERES, NOTAMMENT DES ONGLES**
VERFAHREN UND VORRICHTUNG ZUR BESCHLEUNIGUNG DES WACHSTUMS VON HAUTFORTSÄTZEN, BESONDERS NÄGELN
METHOD AND DEVICE FOR ACCELERATING THE GROWTH OF SKIN APPENDAGES, IN PARTICULAR NAILS

(30) Priorité: 22.06.2004 FR 0406744
(43) Date de publication de la demande: 07.03.2007
(73) Titulaire: Millet Innovation, 26270 Loriol sur Drome (FR)
(72) Inventeur: KOYAZOUNDA, Antoine, F-26000 Valence (FR); MILLET, Jean-Claude, F-26800 Etoile sur Rhone (FR)
(74) Mandataire: Marchand, André
(86) Numéro de dépôt international: PCT/FR2005/001514
(87) Numéro de publication internationale: WO 2006/008373

(56) Documents cités:
- EP-A- 0 179 675
- US-A- 5 181 914
- US-A- 6 042 845
- US-A1- 2002 095 107
- US-B1- 6 727 401

## Description

La présente invention concerne un procédé pour accélérer la croissance des phanères et, plus particulièrement, la croissance des ongles, ainsi qu'un dispositif permettant de mettre en oeuvre ce procédé.

Les phanères sont des productions épidermiques apparentes du corps humain ou animal, qui forment les ongles, les griffes, les poils, la laine, les cheveux, les plumes et les cornes. Ces productions épidermiques sont essentiellement constituées de kératine, une protéine qui résulte de la transformation des cellules de la peau. Dans le cas des ongles, la kératine est fabriquée par les cellules d'une matrice située à la rainure unguéale. Cette matrice se prolonge par un lit sur lequel repose l'ongle, ce lit étant délimité par le sillon péri-unguéal.

En pratique, on estime que les ongles poussent à une vitesse moyenne d'environ 0,1 mm par jour. Il faut environ 4 à 6 mois pour un renouvellement complet des ongles des mains et environ 12 à 18 mois pour un renouvellement complet des ongles des orteils, dont la vitesse de pousse est plus faible que celle des ongles des mains.

Ce temps est excessivement long et il n'est pas possible, à un sujet sain dont l'un des ongles serait par exemple cassé, d'accélérer la repousse de cet ongle.

De même, il n'est pas possible d'accélérer la repousse des ongles de sujets atteints de mycoses unguéales, qui sont des affections très fréquentes ayant notamment pour effet de ralentir voire d'interrompre la croissance des ongles. Certes, ces mycoses unguéales sont traitées localement par l'application de crèmes, ou par la prise de comprimés dont le principe actif est véhiculé par la circulation sanguine jusqu'au lieu de l'infection. Néanmoins, ces crèmes ou comprimés n'ont qu'un effet sur la mycose elle-même et ne favorisent pas la repousse des ongles.

Ainsi, la présente invention vise un procédé et un dispositif permettant d'accélérer la croissance des phanères et notamment des ongles.

Cet objectif est atteint par la prévision d'un procédé comprenant les étapes suivantes : fournir un pansement comprenant un tissu enveloppant ainsi qu'une garniture destinée à être en contact avec au moins un phanère, préparer une base liquide ou visqueuse comprenant un antimicrobien ou un antimycosique, un composé favorisant la pénétration de l'antimicrobien ou de l'antimycosique dans la peau et/ou le phanère, et de l'eau dans une proportion suffisante pour maintenir un degré d'humidité élevé au voisinage du phanère, déposer la base liquide ou visqueuse sur la garniture du pansement, et positionner le pansement comprenant la base liquide ou visqueuse de manière que la garniture soit au contact du phanère.

Le ou les phanères objet du traitement sont ainsi maintenus dans un environnement se caractérisant par un important degré d'humidité. La présence d'un antimicrobien ou antimycosique et d'un composé favorisant la pénétration de l'antimicrobien ou antimycosique dans la peau ou les phanères, ainsi que le degré d'humidité concourent, de manière surprenante, à favoriser la croissance des phanères.

Selon un mode de réalisation, la base liquide ou visqueuse comprend de l'eau dans une proportion supérieure à 90% en poids total de la base liquide ou visqueuse.

Selon un mode de réalisation, le composé favorisant la pénétration de l'antimicrobien ou de l'antimycosique dans la peau et/ou le phanère est du polyéthylène glycol.

Selon un mode de réalisation, le polyéthylène glycol a un poids moléculaire supérieur ou égal à 6000 et, préférentiellement, de l'ordre de 8000.

Selon un mode de réalisation, la base liquide ou visqueuse comprend en outre un polysaccharide.

Selon un mode de réalisation, le polysaccharide est un scléroglucane.

Selon un mode de réalisation, la base liquide ou visqueuse comprend en outre un alcool.

Selon un mode de réalisation, l'antimicrobien ou antimycosique est le thymol.

La présente invention concerne également un dispositif pour accélérer la croissance des phanères, en particulier des ongles, comprenant un pansement sensiblement imperméable à l'eau comprenant un tissu enveloppant, une garniture du pansement destinée à être en contact avec au moins un phanère, et une base liquide ou visqueuse en contact avec la garniture et comprenant un antimicrobien ou un antimycosique, un composé favorisant la pénétration de l'antimicrobien ou de l'antimycosique dans la peau et/ou le phanère, et de l'eau dans une proportion suffisante pour maintenir un degré d'humidité élevé au voisinage du phanère.

Selon un mode de réalisation, le pansement est occlusif.

Selon un mode de réalisation, le pansement forme un doigtier.

Selon un mode de réalisation, le tissu enveloppant est un polyamide élasthanne et la garniture est un gel silicone.

Selon un mode de réalisation, la base liquide ou visqueuse comprend de l'eau dans une proportion supérieure à 90% en poids total de la base liquide ou visqueuse.

Selon un mode de réalisation, la base liquide ou visqueuse comprend en outre un polysaccharide.

Selon un mode de réalisation, le polysaccharide est un scléroglucane.

Selon un mode de réalisation, la base liquide ou visqueuse comprend en outre un alcool.

Selon un mode de réalisation, l'antimicrobien ou l'antimycosique est le thymol.

Selon un mode de réalisation, le composé favorisant la pénétration de l'antimicrobien ou de l'antimycosique dans la peau et/ou le phanère est le polyéthylène glycol.

Selon un mode de réalisation, le polyéthylène glycol a un poids moléculaire supérieur ou égal à 6000 et, préférentiellement, de l'ordre de 8000.

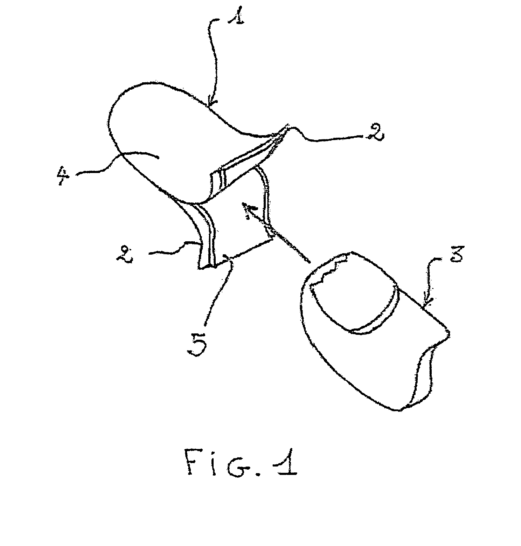

L'invention sera mieux comprise à la lecture de la description suivante d'un exemple de réalisation de l'invention, faite à titre non limitatif en relation avec la figure 1.

La figure 1 représente schématiquement un dispositif selon l'invention prévu pour être appliqué à l'extrémité d'un doigt ou d'un orteil, par exemple le gros orteil 3. Le dispositif prend ici la forme d'un pansement 1 occlusif, sensiblement imperméable à l'eau et autres liquides, mais autorisant éventuellement des échanges gazeux selon le mode de réalisation choisi. Le pansement forme ici un doigtier enveloppant, qui vient couvrir la totalité de la dernière phalange de l'orteil 3. Ce doigtier est formé d'une feuille rabattue puis cousue ou soudée le long de ses bords sur une partie de sa longueur, ou de deux feuilles superposées puis cousues ou soudées le long de leurs bords. L'extrémité distale du doigtier n'est pas cousue ou soudée et présente ainsi deux ailettes 2 qui facilitent la mise en place du pansement sur l'orteil 3 et peuvent être découpées afin d'adapter la longueur du doigtier à celle de l'orteil. La ligne de couture ou de soudage ou, plus généralement, de raccordement entre les bords de la feuille ou des feuilles formant le doigtier, peut ne pas être totalement étanche à la base liquide ou visqueuse décrite dans ce qui suit, autorisant ainsi les échanges gazeux.

De préférence, la feuille comprend un tissu 4 extérieur enveloppant et une garniture 5 intérieure. Le tissu 4 comprend un mélange de fibres élastiques et de fibres thermoplastiques. Les fibres élastiques sont de préférence constituées d'élasthanne et les fibres thermoplastiques sont constituées de polyamide. Le tissu comprend par exemple un mélange de fibres de polyamide et d'élasthanne en proportions respectives de 70% et de 30%.

La garniture 5 est destinée à être directement en contact avec l'extrémité du doigt ou de l'orteil. Elle est par exemple formée d'un gel de silicone réputé pour sa compatibilité avec la peau. Il s'agit par exemple du gel de silicone commercialisé par la demanderesse sous la dénomination EPITHELIUM 26 (TM), qui reproduit les caractéristiques du capiton plantaire, ainsi que les gels commercialisés sous les dénominations EPITHELIUM 27 (TM) et EPITHELIUM 27+ (TM). Ces gels limitent considérablement les phénomènes d'irritation qui peuvent résulter de frottements en milieu humide.

Le dispositif selon l'invention comporte en outre une base liquide ou visqueuse.

Cette base liquide ou visqueuse est par exemple un gel ou une crème. La base comprend un antimicrobien ou un antimycosique, un composé favorisant la pénétration de l'antimicrobien ou de l'antimycosique dans la peau et/ou l'ongle, un polysaccharide, de l'alcool et de l'eau dans une proportion importante, de préférence supérieure à 90% en poids total de la base.

L'antimicrobien est un antibactérien ou un antimycosique. Il s'agit par exemple d'un antibactérien qui peut être d'origine naturelle, par exemple le thymol, dont la dissolution est favorisée par la présence de l'alcool dans la base.

Le composé favorisant la pénétration de l'antimicrobien ou de l'antimycosique dans la peau et/ou l'ongle lui-même, ramolli par la présence d'eau, est par exemple le polyéthylène glycol et préférentiellement un polyéthylène glycol (PEG) de haut poids moléculaire, supérieur ou égal à 6000, de préférence de l'ordre de 8000, qui montre une grande stabilité dans le temps.

Le polysaccharide est notamment une gomme sclérotium obtenue par fermentation d'une moisissure, *Sclerotium rolfsii,* dans un milieu enrichi en glucose. Ce polysaccharide forme, en présence d'eau, un gel transparent.

Bien entendu, d'autres composés peuvent être ajoutés à la base liquide ou visqueuse selon l'invention. Il s'agit notamment de composés destinés à apporter des nutriments à la peau tels que la vitamine A.

Pour la fabrication de la base liquide ou visqueuse, on procède par exemple de la manière suivante. On mélange tout d'abord 5 grammes de polyéthylène glycol de masse moléculaire voisine de 8000 dans 20 grammes d'alcool, jusqu'à obtention d'un liquide homogène formant un sous-ensemble A. On ajoute ensuite 1 gramme de thymol à ce sous-ensemble et on mélange jusqu'à dissolution complète pour obtenir un sous-ensemble B. On prépare alors une solution de polysaccharide, par mélange de 0,8% en poids de gomme sclérotium dans de l'eau en vue d'obtenir un sous-ensemble C. Finalement, on mélange le sous-ensemble B dans le sous-ensemble C selon des proportions 1 x B pour 99 x C, ce qui donne un gel relativement liquide et transparent, constituant la base liquide ou visqueuse.

Pour la mise en oeuvre de l'invention, on place une noisette de gel dans le fond du doigtier et on positionne ce doigtier sur l'orteil de manière qu'il couvre la totalité de l'extrémité de l'orteil.

On crée ainsi immédiatement un environnement qui est à la fois humide à saturation (au-delà de 80% d'humidité), anti-infectieux et nourrissant pour l'ongle. Le doigtier est porté pendant une période la plus longue possible en fonction des contraintes diverses des usagers, mais pas moins de 12 heures par jour. L'environnement à la fois humide, anti-infectieux et nourrissant pour l'ongle, est ainsi maintenu dans le temps.

Il apparaît que le dispositif selon l'invention produit un effet sur l'ongle dans un délai de quelques jours, en pratique de 15 à 30 jours, selon le temps d'utilisation journalier du dispositif. Au terme de ce délai, on observe une vitesse de croissance de l'ongle environ trois fois supérieure à celle qui est observée dans les circonstances habituelles pour un sujet sain, ce qui est en rupture totale avec ce qui est connu à ce jour. Dans le cas particulier d'un ongle d'orteil, on constate que le temps de renouvellement complet de l'ongle selon le procédé de l'invention est ramené à 4 mois environ au lieu de 12 à 18 mois pour un sujet sain.

Le pansement selon l'invention peut bien entendu être conçu pour recevoir plusieurs doigts ou orteils, pour une application collective du procédé à plusieurs ongles.

On notera que l'exposition des orteils à un milieu humide est réputée favoriser l'implantation et le développement des champignons qui sont responsables des mycoses. L'invention va à l'encontre de ce préjugé et en créant un environnement humide qui ne présente pas un tel inconvénient, même dans le cas ou le sujet présente une onychomycose. D'ailleurs, dans un tel cas, l'onychomycose disparaît progressivement par l'application du dispositif selon l'invention.

Il apparaîtra clairement à l'homme de l'art que le dispositif selon l'invention est susceptible de diverses variantes de réalisation, notamment en ce qui concerne le support recevant la base liquide ou visqueuse permettant de maintenir pendant un certain temps un degré d'humidité élevé. C'est le cas par exemple des pansements qui associent, sous une membrane adhésive en polyuréthanne, des hydrocolloïdes.

## Revendications

1. Dispositif pour accélérer la croissance des phanères, en particulier des ongles, comprenant :
- un pansement sensiblement imperméable à l'eau comprenant un tissu enveloppant, et
- une garniture du pansement destinée à être en contact avec au moins un phanère,
**caractérisé en ce qu'**il comporte en outre :
- une base liquide ou visqueuse à placer dans le pansement de manière que la base liquide ou visqueuse soit en contact avec le phanère, la base liquide ou visqueuse comprenant :
- un antimicrobien ou un antimycosique,
- un composé favorisant la pénétration de l'antimicrobien ou de l'antimycosique dans la peau et/ou le phanère,
- un polysaccharide, et
- de l'eau dans une proportion suffisante pour maintenir au voisinage du phanère un degré d'humidité élevé favorisant la croissance du phanère.

2. Dispositif selon la revendication 1, dans lequel le pansement est occlusif.

3. Dispositif selon l'une des revendications 1 ou 2, pour la croissance des ongles, dans lequel le pansement forme un doigtier.

4. Dispositif selon l'une des revendications 1, 2 ou 3, dans lequel le tissu enveloppant est un polyamide élasthanne et la garniture est un gel silicone.

5. Dispositif selon l'une des revendications 1 à 4, dans lequel la base liquide ou visqueuse comprend de l'eau dans une proportion supérieure à 90% en poids total de la base liquide ou visqueuse.

6. Dispositif selon l'une des revendications 1 à 5, dans lequel le polysaccharide est un scléroglucane.

7. Dispositif selon l'une des revendications 1 à 6, dans lequel la base liquide ou visqueuse comprend en outre un alcool.

8. Dispositif selon l'une des revendications 1 à 7, dans lequel l'anti-microbien ou l'antimycosique est le thymol.

9. Dispositif selon l'une des revendications 1 à 8, dans lequel le composé favorisant la pénétration de l'antimicrobien ou de l'antimycosique dans la peau et/ou le phanère est le polyéthylène glycol.

10. Dispositif selon la revendication 9, dans lequel le polyéthylène glycol a un poids moléculaire supérieur ou égal à 6000 et, préférentiellement, de l'ordre de 8000.

11. Procédé pour accélérer la croissance des phanères, en particulier des ongles, **caractérisé en ce qu'**il comporte les étapes suivantes :
fournir un pansement comprenant un tissu enveloppant ainsi qu'une garniture destinée à être en contact avec au moins un phanère,
préparer une base liquide ou visqueuse comprenant un antimicrobien ou un antimycosique, un composé favorisant la pénétration de l'antimicrobien ou de l'antimycosique dans la peau et/ou le phanère, et de l'eau dans une proportion suffisante pour maintenir un degré d'humidité élevé au voisinage du phanère,
déposer la base liquide ou visqueuse sur la garniture du pansement, et
positionner le pansement comprenant la base liquide ou visqueuse de manière que la garniture soit au contact du phanère.

12. Procédé selon la revendication 11, dans lequel la base liquide ou visqueuse comprend de l'eau dans une proportion supérieure à 90% en poids total de la base liquide ou visqueuse.

13. Procédé selon l'une des revendications 11 et 12, dans lequel le composé favorisant la pénétration de l'antimicrobien ou de l'antimycosique dans la peau et/ou le phanère est du polyéthylène glycol.

14. Procédé selon la revendication 13, dans lequel le polyéthylène glycol a un poids moléculaire supérieur ou égal à 6000 et, préférentiellement, de l'ordre de 8000.

15. Procédé selon l'une des revendications 11 à 14, dans lequel la base liquide ou visqueuse comprend en outre un polysaccharide.

16. Procédé selon la revendication 15, dans lequel le polysaccharide est un scléroglucane.

17. Procédé selon l'une des revendications 11 à 16, dans lequel la base liquide ou visqueuse comprend en outre un alcool.

18. Procédé selon l'une des revendications 11 à 17, dans lequel l'anti-microbien ou antimycosique est le thymol.

## Claims

1. Device for accelerating the growth of skin appendages, in particular nails, comprising:
- a bandage substantially waterproof comprising an enveloping cloth, and
- a bandage stuffing intended to be in contact with at least one skin appendage, further comprising:
- a liquid or viscous base to be put into the bandage so that the liquid or viscous base is in contact with the skin appendage, the liquid or viscous base comprising:
- an antimicrobial or an antimycosic
- a compound helping the penetration of the antimicrobial or antimycosic into the skin and/or the skin appendage,
- a polysaccharide, and
- water in a sufficient proportion to maintain near the skin appendage a high degree of humidity helping the growth of the skin appendage.

2. Device according to claim 1, wherein the bandage is occlusive.

3. Device according to one of claims 1 or 2, for the growth of nails, wherein the bandage forms a finger cot.

4. Device according to one of claims 1, 2 or 3, wherein the enveloping cloth is a spandex-polyamide and the stuffing is a silicon gel.

5. Device according to one of claims 1 to 4, wherein the liquid or viscous base comprises water in a proportion greater than 90% of total weight of the liquid or viscous base.

6. Device according to one of claims 1 to 5, wherein the polysaccharide is a scleroglucane.

7. Device according to one of claims 1 to 6, wherein the liquid or viscous base further comprises an alcohol.

8. Device according to one of claims 1 to 7, wherein the antimicrobial or the antimycosic is the thymol.

9. Device according to one of claims 1 to 8, wherein the compound helping the penetration of the antimicrobial or antimycosic into the skin and/or the skin appendage is the polyethylene glycol.

10. Device according to claim 9, wherein the polyethylene glycol has a molecular mass superior or equal to 6,000 and, preferably around 8,000.

11. Method for accelerating the growth of skin appendages, in particular nails, comprising:
supplying a bandage comprising an enveloping cloth as well as a stuffing intended to be in contact with at least one skin appendage,
preparing a liquid or viscous base comprising an antimicrobial or an antimycosic, a compound helping the penetration of the antimicrobial or antimycosic into the skin and/or the skin appendage, and water in a proportion sufficient to maintain a high degree of humidity near the skin appendage,
laying the liquid or viscous base down on the bandage stuffing, and
positioning the bandage comprising the liquid or viscous base so that the stuffing is in contact with the skin appendage.

12. Method according to claim 11, wherein the liquid or viscous base comprises water in a proportion greater than 90% of total weight of the liquid or viscous base.

13. Method according to one of claims 11 and 12, wherein the compound helping the penetration of the antimicrobial or the antimycosic into the skin and/or the skin appendage is polyethylene glycol.

14. Method according to claim 13, wherein the polyethylene glycol has a molecular mass superior or equal to 6,000, and preferably around 8,000.

15. Method according to one of claims 11 to 14, wherein the liquid or viscous base further comprises a polysaccharide.

16. Method according to claim 15, wherein the polysaccharide is a scleroglucane.

17. Method according to one of claims 11 to 16, wherein the liquid or viscous base further comprises an alcohol.

18. Method according to one of claims 11 to 17, wherein the antimicrobial or the antimycosic is the thymol.

## Patentansprüche

1. Vorrichtung zur Beschleunigung des Wachstums der Phanere, insbesondere der Nägel, umfassend:
- einen im Wesentlichen wasserundurchlässigen Verband, umfassend einen Umhüllungsstoff, und
- eine Verbandeinlage, die dazu bestimmt ist, mit mindestens einem der Phanere in Kontakt zu sein,
**dadurch gekennzeichnet, dass** sie ferner umfasst:
- eine flüssige oder viskose Basis, die im Verband anzuordnen ist, so dass die flüssige oder viskose Basis mit den Phaneren in Kontakt ist, wobei die flüssige oder viskose Basis umfasst:
- ein Mikroben- oder Pilzschutzmittel,
- eine Verbindung, die das Eindringen des Mikroben- oder Pilzschutzmittels in die Haut und/oder die Phanere begünstigt,
- ein Polysacharid, und
- Wasser in einem ausreichenden Verhältnis, um in der Nähe der Phanere einen hohen Feuchtigkeitsgrad aufrecht zu erhalten, der das Wachstum der Phanere begünstigt.

2. Vorrichtung nach Anspruch 1, bei der der Verband verschließend ist.

3. Vorrichtung nach einem der Ansprüche 1 oder 2 für das Wachstum der Nägel, bei der der Verband einen Fingerling bildet.

4. Vorrichtung nach einem der Ansprüche 1, 2 oder 3, bei der der Umhüllungsstoff ein Elasthanpolyamid und die Einlage ein Silikongel ist.

5. Vorrichtung nach einem der Ansprüche 1 bis 4, bei der die flüssige oder viskose Basis Wasser in einem Verhältnis von mehr als 90 % des Gesamtgewichts der flüssigen oder viskosen Basis umfasst.

6. Vorrichtung nach einem der Ansprüche 1 bis 5, bei der das Polysacharid ein Skleroglukan ist.

7. Vorrichtung nach einem der Ansprüche 1 bis 6, bei der die flüssige oder viskose Basis ferner einen Alkohol umfasst.

8. Vorrichtung nach einem der Ansprüche 1 bis 7, bei der das Mikroben- oder Pilzschutzmittel Thymol ist.

9. Vorrichtung nach einem der Ansprüche 1 bis 8, bei der die Verbindung, die das Eindringen des Mikroben- oder Pilzschutzmittels in die Haut und/oder die Phanere begünstigt, Glykolpolyethylen ist.

10. Vorrichtung nach Anspruch 9, bei der das Glykolpolyethylen ein Molekulargewicht größer oder gleich 6000 und vorzugsweise von ungefähr 8000 hat.

11. Verfahren zur Beschleunigung des Wachstums der Phanere, insbesondere der Nägel, **dadurch gekennzeichnet, dass** es die folgenden Schritte umfasst:
- Bereitstellung eines Verbandes, umfassend einen Umhüllungsstoff sowie eine Einlage, die dazu bestimmt ist, mit mindestens einem der Phanere in Kontakt zu sein,
- Vorbereitung einer flüssigen oder viskosen Basis, umfassend ein Mikroben- oder Pilzschutzmittel, eine Verbindung, die das Eindringen des Mikroben-oder Pilzschutzmittels in die Haut und/oder die Phanere begünstigt, und Wasser in einem ausreichenden Verhältnis, um einen hohen Feuchtigkeitsgrad in der Nähe der Phanere aufrecht zu erhalten,
- Aufbringen der flüssigen oder viskosen Basis auf die Einlage des Verbandes, und
- Anbringen des Verbandes, umfassend die flüssige oder viskose Basis, derart, dass die Einlage mit den Phaneren in Kontakt ist.

12. Verfahren nach Anspruch 11, bei dem die flüssige oder viskose Basis Wasser in einem Verhältnis von mehr als 90 % des Gesamtgewichts der flüssigen oder viskosen Basis umfasst.

13. Verfahren nach einem der Ansprüche 11 und 12, bei dem die Verbindung, die das Eindringen des Mikroben- oder Pilzschutzmittels in die Haut und/oder die Phanere begünstigt, Glykolpolyethylen ist.

14. Verfahren nach Anspruch 13, bei dem das Glykolpolyethylen ein Molekulargewicht größer oder gleich 6000 und vorzugsweise von ungefähr 8000 hat.

15. Verfahren nach einem der Ansprüche 11 bis 14, bei dem die flüssige oder viskose Basis ferner ein Polysacharid umfasst.

16. Verfahren nach Anspruch 15, bei dem das Polysacharid ein Skleroglukan ist.

17. Verfahren nach einem der Ansprüche 11 bis 16, bei dem die flüssige oder viskose Basis ferner einen Alkohol umfasst.

18. Verfahren nach einem der Ansprüche 11 bis 17, bei dem das Mikroben- oder Pilzschutzmittel Thymol ist.
